# EUROPÄISCHE PATENTANMELDUNG

(11) **EP 1 522 260 A1**
(43) Veröffentlichungstag der Anmeldung: **13.04.2005**
(21) Anmeldenummer: 03014772.2
(22) Anmeldetag: 27.06.2003
(51) Int. Cl.: A61B 5/15

(54) **Vorrichtung zur Blutabnahme und gleichzeitigen quantitativen Bestimmung von Analyten im Blut**

(71) Anmelder: Ehrfeld Mikrotechnik AG, 55234 Wendelsheim (DE); Novartis AG, 4056 Basel (CH)
(72) Erfinder: Die Erfindernennung liegt noch nicht vor

(57) **Zusammenfassung**

Es wird eine Vorrichtung zur Blutabnahme und gleichzeitigen Durchführung der Nachweisreaktion von Analyten im Blut beschrieben, die aus einem Handteil (1) und einer Sensoreinheit (2) besteht, wobei
- a): die Sensoreinheit (2) eine Mikronadel (3) enthält, die in einer elastischen Membran (4) befestigt bei Ausübung von Druck bzw. Kräften auf die Membran in die Haut eindringt und beim Nachlassen des Druckes oder der Kräfte die Einstichstelle unter Ausbildung eines Unterdrucks in der Sensoreinheit wieder verlässt, wobei durch Blut in die Sensoreinheit eingesaugt wird, welches dort mit einem Sensorsystem eine physikalische oder chemische Eigenschaftsänderung des Sensorsystems bewirkt und
- b): das Handteil pneumatisch, mechanisch oder elektromechanisch auf die Membran der Sensoreinheit einwirkt.
Es wird außerdem ein Verfahren zur quantitativen Bestimmung von Analyten im Blut mit Hilfe der vorstehend gennanten Vorrichtung beschrieben.

## Beschreibung

Gegenstand der Erfindung ist eine Vorrichtung, mit der die Entnahme einer Blutprobe sowie die quantitative Bestimmung eines oder mehrerer Blutinhaltsstoffe in einem Arbeitsschritt zusammengefasst werden kann.

Es sind bereits zahlreiche Geräte zum Durchstechen der menschlichen Haut und zum Sammeln von Blut durch Ansaugen bekannt. So ist beispielsweise in der europäischen Patentschrift 0 622 046 B1 eine Saug- oder Vakuumvorrichtung zur Blutentnahme vorgesehen, die die menschliche Haut bei der Entnahme einer vorbestimmten Menge Blut mit hoher Geschwindigkeit durchstechen und ein Aufmischen des gesammelten Blutes verhindern kann.

Aus der internationalen Patentanmeldung WO 01/28423 sind außerdem eine Stechhilfe und ein Verfahren zur Entnahme von Blut aus Geweben bekannt, bei dem die Eindringtiefe der Nadel kontrolliert und die Blutmenge durch Anwendung von Druck stimuliert wird.

Derartige Vorrichtungen haben den Vorteil, dass eine Blutentnahme auch dann möglich ist, wenn die Dichte der feinen Blutgefäße in der Haut nur gering ist, weil das Blut durch ein Vakuum angesaugt oder abgezogen wird. Nachteilig bei den bekannten Blutentnahmevorrichtungen ist jedoch, dass das entnommene Blut anschließend auf einen Teststreifen oder in ein Messgerät übertragen werden muss, womit Fehlerquellen entstehen und komplizierte und zeitaufwendige Arbeitsabläufe erforderlich sind. Hinzu kommt, dass die Blutentnahme nach dem herkömmlichen Verfahren fast immer mit Schmerzen verbunden ist und vom Patienten als unangenehm empfunden wird.

Es stellte sich deshalb die Aufgabe, das für Blutuntersuchungen erforderliche Blutvolumen erheblich zu reduzieren und die quantitative Bestimmung des im Blut nachzuweisenden Analyten frei von etwaigen Messfehlern und erheblich schneller dadurch durchzuführen, dass Blutentnahme und Durchführung der Nachweisreaktion sowie ggf. Blutuntersuchung in einem einzigen Arbeitsschritt durchgeführt werden. Außerdem stellte sich die Aufgabe, durch den Einsatz von besonders dünnen Mikronadeln das subjektive Schmerzempfinden des Patienten erheblich zu vermindern.

Gelöst wird diese Aufgabe durch eine Vorrichtung zur Blutabnahme und gleichzeitigen Durchführung der Nachweisreaktion von Analyten im Blut, die aus einem Handteil 1 und einer Sensoreinheit 2 besteht, wobei
a) die Sensoreinheit 2 eine Mikronadel 3 enthält, die in einer elastischen Membran 4 befestigt bei Ausübung von Druck bzw. Kräften auf die Membran in die Haut eindringt und beim Nachlassen des Druckes oder der Kräfte die Einstichstelle unter Ausbildung eines Unterdrucks in der Sensoreinheit wieder verlässt, wodurch Blut in die Sensoreinheit gesaugt wird, welches dort mit einem Sensorsystem eine physikalisch oder chemische Eigenschaftsänderung des Sensorsystems bewirkt.
b) das Handteil pneumatisch, mechanisch oder elektromechanisch auf die Membran der Sensoreinheit einwirkt

Die erfindungsgemäß verwendete Sensoreinheit erlaubt nicht nur die Bestimmung eines einzigen Analyten, zum Beispiel die Bestimmung der Glukose im Blut, sondern kann je nach dem eingebauten Sensorsystem auch für andere Analyten und sogar zur gleichzeitigen Bestimmung mehrerer verschiedener Analyten eingesetzt werden, indem in die Sensoreinheit mehrere unterschiedliche Sensorsysteme eingefügt werden. In einer fortgeschrittenen Ausführungsform kann die aus Handteil und Sensoreinheit bestehende Vorrichtung unmittelbar in ein Analysesystem integriert werden, mit der beispielsweise Änderungen der Farbe, des Fluoreszenzverhaltens, der elektrischen Leitfähigkeit, oder sonstiger chemischer oder physikalischer Größen registriert werden.

Die erfindungsgemäße Sensoreinheit ist in der Regel zum einmaligen Gebrauch bestimmt, kann aber auch bei Einfügung mehrerer Sensoreinheiten, z. B. auf einer drehbaren Zuführungseinheit für das Blut, mehrmals für die Bestimmung des gleichen Analyten verwendet werden. Sie kann mit dem Handteil verbunden und von ihm nach jeder erfolgten Messung auch wieder leicht entfernt werden.

Die erfindungsgemäße Vorrichtung wird durch die folgenden Darstellungen im Einzelnen verdeutlicht. Diese Darstellungen sind nur beispielhaft und sollten nicht als Einschränkung der technischen Ausführungsform aufgefasst werden.

Es zeigen:
- Fig. 1: einen Überblick über die erfindungsgemäße Vorrichtung bestehend aus einem Handteil 1 und einer Sensoreinheit 2;
- Fig. 2: den Ablauf der Blutentnahme und der Durchführung der Nachweisreaktion sowie der externen Bestimmung des Analyten in acht Teilschritten;
- Fig. 2a: den Ausgangszustand;
- Fig. 2b: die Sensoreinheit ist aufgeschnappt;
- Fig. 2c: das Spannen der Feder;
- Fig. 2d: das Aufsetzen auf die Haut;
- Fig. 2e: die Stechphase;
- Fig. 2f: die Saugphase;
- Fig. 2g: die Messung;
- Fig. 2h: den Abwurf der verbrauchten Sensoreinheit.
- Fig. 3 Fig. 3a: das Handteil mit Sensoreinheit;
das Handteil, bestehend aus zwei Hülsenteilen mit unterschiedlichen Funktionen mit aufgesetzter Sensoreinheit;
- Fig. 3b: Schnittdarstellung des Handteils mit aufgesetzter Sensoreinheit;
- Fig. 4: eine Detailansicht für die Einschnapp-/Abwurfvorrichtung;
- Fig. 5: eine Detailansicht für den Auslösemechanismus;
- Fig. 6a-e: Schnitte durch mehrere, unterschiedlich konstruierte Sensoreinheiten;
- Fig. 7: eine für mehrere verschiedene Analyten geeignete Sensoreinheit;
- Fig. 8: eine Sensoreinheit mit einer drehbaren Zuführung des Blutes, durch die eine mehrmalige Messung des gleichen Analyten, oder auch verschiedener Analyten zu verschiedenen Zeiten, möglich ist;
- Fig.9: eine erfindungsgemäße Vorrichtung, bei der die Einstechtiefe durch Änderung des Luftdruckes über der elastischen Membran variabel eingestellt werden kann;
- Fig. 10 und 11: Sensoreinheiten mit Vorrichtungen zur Filtration oder Vorratsbehältern für Reagenzien oder Spüllösungen;
- Fig. 12: eine Sensoreinheit mit aufgesetztem Messgerät, wobei Reagenzien oder Spüllösungen aus entsprechenden Vorratsgefäßen aus dem Messgerät zudosiert werden können.
- Fig. 13: eine Sensoreinheit mit aufgestecktem Heizblock zur thermischen Vorbehandlung.

Die Funktionsweise der erfindungsgemäßen Vorrichtung wird in folgender Weise näher beschrieben:

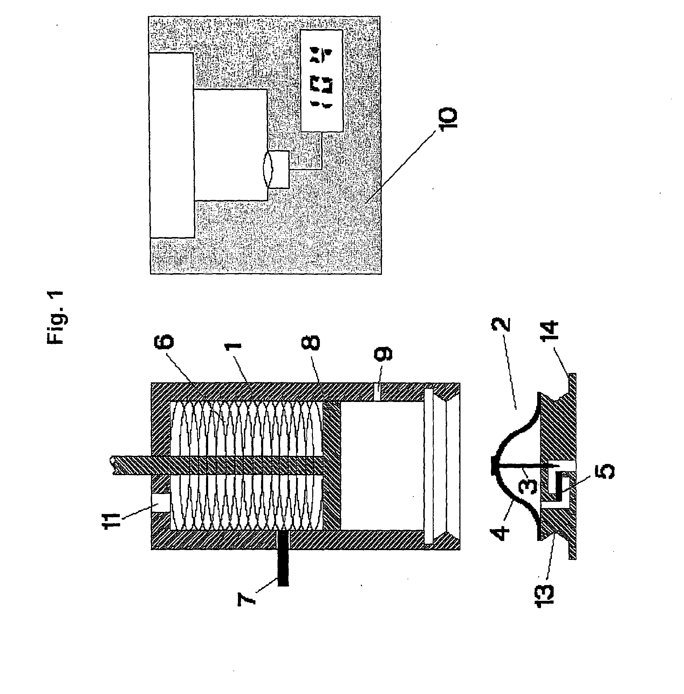

Fig. 1 zeigt eine spezielle Ausführung der erfindungsgemäßen Vorrichtung, die die prinzipielle Funktionsweise verdeutlicht. Die Vorrichtung besteht aus einem wieder zu verwendenden Handteil 1, in dem die Funktionseinheiten für den Antrieb untergebracht sind, und aus einer Sensoreinheit 2, die eine Mikronadel 3 in einer elastischen Membran 4 und ein Sensorsystem 5 enthält. Die vornehmlich zum einmaligen Gebrauch bestimmte Sensoreinheit 2 kann von unten mit dem Handteil verbunden werden, z. B. durch einen Schnappverschluss 13 bzw. durch eine Abwurfkante 14 wieder entfernt werden. Das Handteil enthält den Antriebsteil zum Aufbau des Druckes, der auf die Membran einwirkt und diese bewegt. Fig. 1 zeigt eine Ausführungsform des Handteils zum Betätigen der Membran, bestehend aus Feder 6, Kolben 8, Druckausgleichsöffnung unten 9, Druckausgleichsöffnung oben 11 und Auslöser 7. Die quantitative Auswertung wird vornehmlich mit einem externen Handmessgerät 10 vorgenommen.

### Ablauf der Blutentnahme und der quantitativen Messung

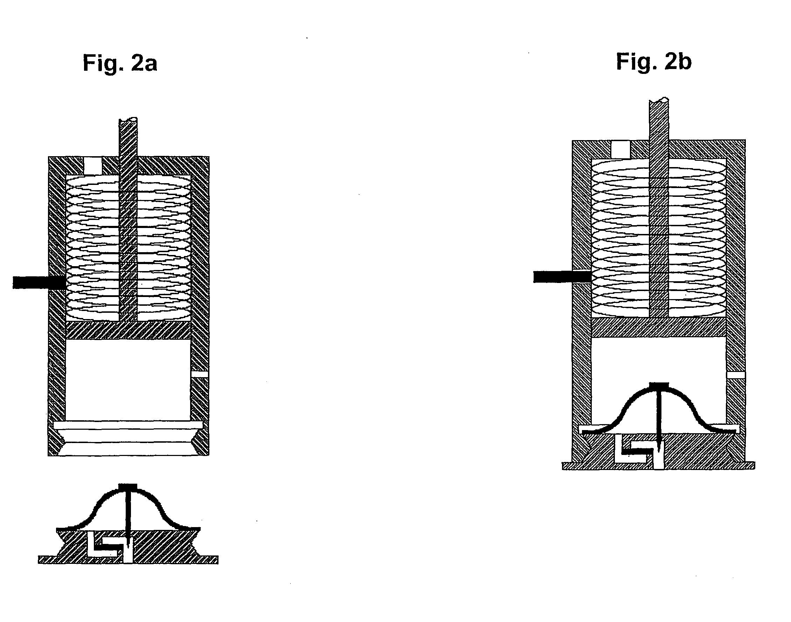

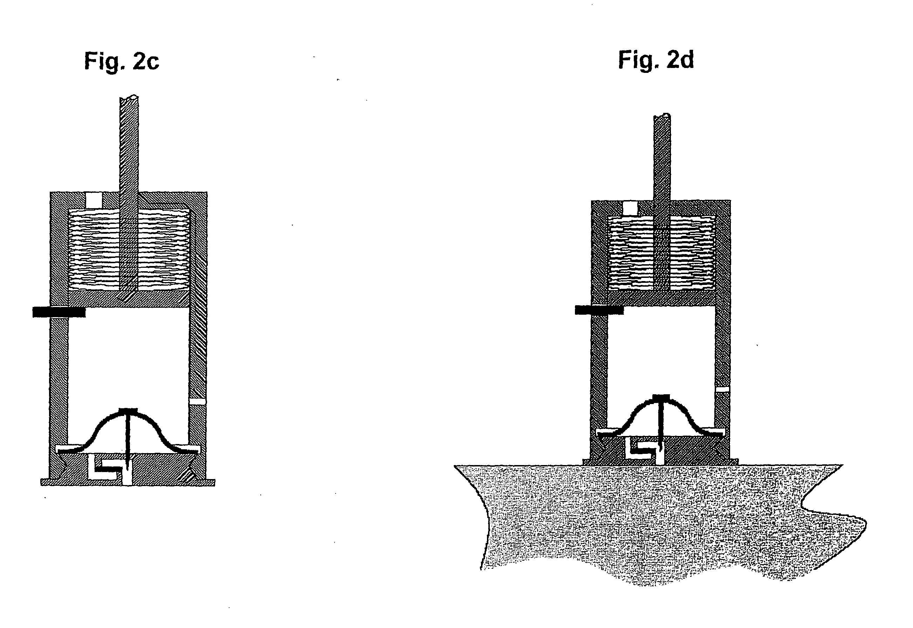

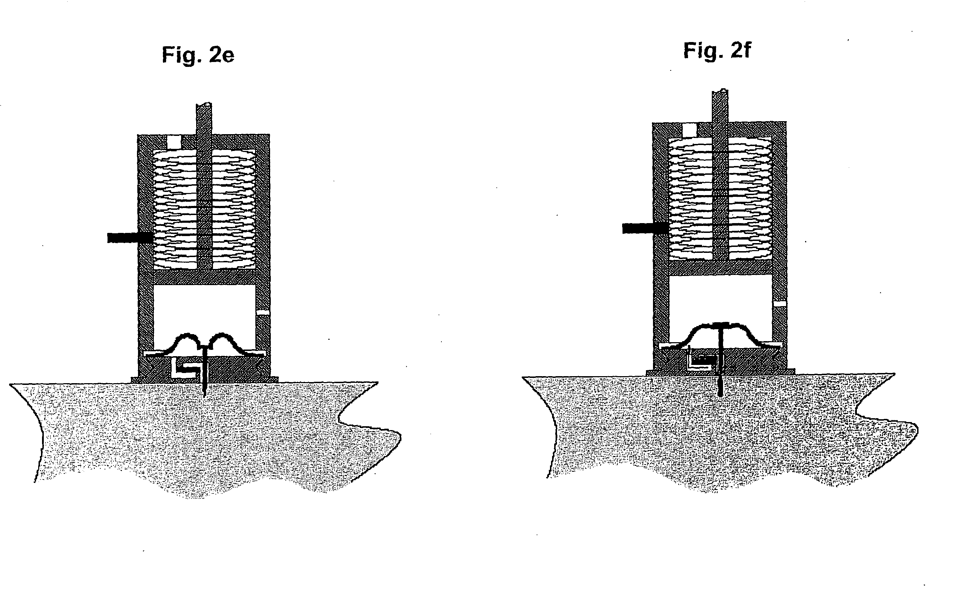

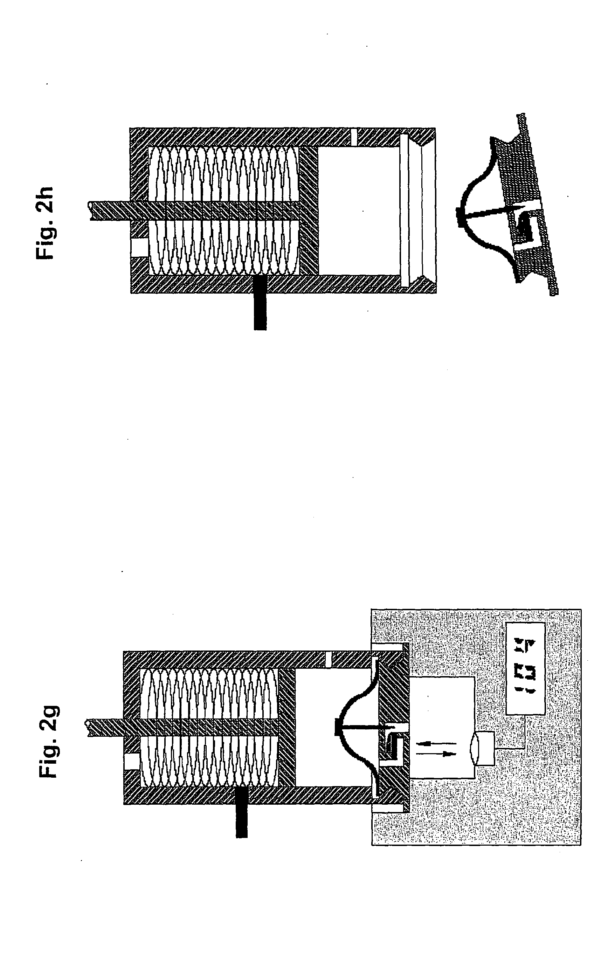

Der Ablauf der Blutentnahme und der quantitativen Messung ist im Überblick in Fig.2 gezeigt.

### Ausgangszustand, Fig. 2a

Fig. 2a zeigt den Ausgangszustand mit dem wieder zu verwendenden Handteil und der Sensoreinheit, welche vornehmlich in einer sterilen Verpackung untergebracht und zum einmaligen Gebrauch bestimmt ist.

### Sensoreinheit ist aufgeschnappt, Fig. 2b

Mittels einer Haltevorrichtung, z. B. mit einem Schnappverschluss, wird die Sensoreinheit vom Handteil aufgenommen.

### Spannen der Feder, Fig. 2c

Der Kolben im Handteil wird nach oben bewegt und die Feder gespannt. Ein am Umfang des Handteils befindlicher Auslöser sperrt die Feder davor sich zu entspannen. Die Druckausgleichsöffnungen be- und entlüften den Innenraum des Handteils.

### Aufsetzen auf Haut, Fig. 2d

Die im Handteil eingesetzte Sensoreinheit wird auf die Hautoberfläche aufgesetzt.

### Stechphase, Fig. 2e

Durch Betätigung des Auslösers wird die Feder entspannt und der Kolben bewegt sich nach unten. Zwischen Kolben und der elastischen Membran wird durch die schnelle Bewegung des Kolbens ein komprimierter Luftraum aufgebaut. Im oberen Handteil kann Luft durch die große obere Druckausgleichsöffnung eindringen. Die elastische Membran verformt sich und die Mikronadel sticht in die Haut. Die Luft innerhalb der Sensoreinheit wird durch seitliche Schlitze nach außen geleitet. Diese Schlitze können auch als Öffnungen mit beliebigem Querschnitt ausgeführt sein und Schließelemente enthalten, die eine Ventilfunktion aufweisen und sich beispielsweise erst nach Überschreiten einer vorgegebenen Druckdifferenz öffnen.

### Saugphase, Fig. 2f

Durch die kleine untere Druckausgleichsöffnung entweicht die komprimierte Luft. Nach Erreichen der maximalen Stechtiefe entspannt sich die elastische Membran wieder und bewegt sich in den Ausgangszustand zurück. Dabei wird ein Unterdruck innerhalb der Sensoreinheit aufgebaut und Blut an das Sensorsystem geleitet. Eine chemische, biochemische oder physikalische Reaktion findet an diesem Sensorsystem statt. Die Folge davon ist eine physikalische oder chemische Eigenschaftsänderung des Sensorsystems.

### Messung, Fig. 2g

Nach Ablauf der Saugphase wird die Sensoreinheit, üblicherweise mit dem Handteil verbunden, auf ein Mess- und Auswertegerät, vorzugsweise ein Handmessgerät, aufgesteckt. Ein oder mehrere Messwerte werden angezeigt.

### Abwurf, Fig. 2h

Nach Beendigung der Messung wird die Sensoreinheit entfernt oder kann für weitere Bluttests verwendet werden. In der Regel wird die Sensoreinheit mittels einer Abwurfmöglichkeit, wie beispielsweise einer Abwurfkante, vom Handteil entfernt. Das Handteil ist jetzt durch Aufstecken einer neuen Sensoreinheit zum Gebrauch für eine weitere Messung einsatzbereit.

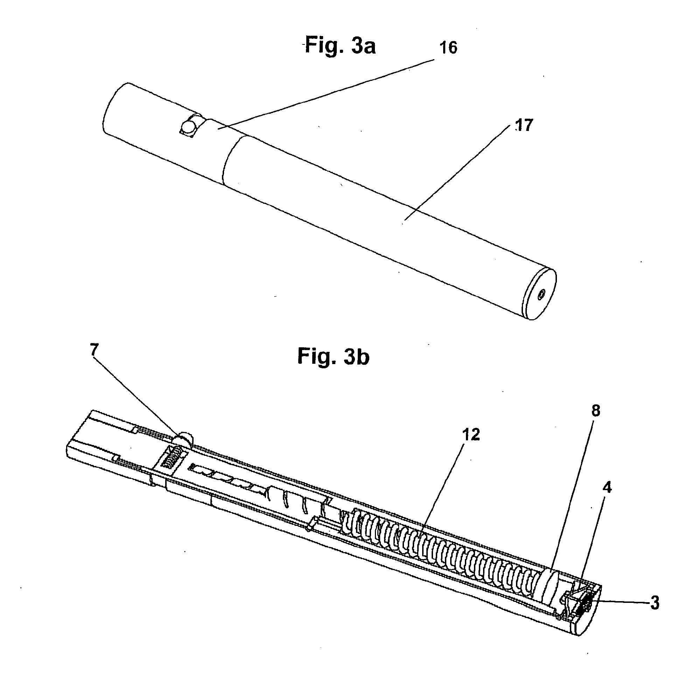

Fig. 3a zeigt eine Zweiteilung der Hülse, wobei durch Drehen des oberen Hülsenteils 16 die Gewindespindel mit der Feder 12 gespannt und gleichzeitig elektrische Energie durch ein entsprechendes Piezoelement gewonnen werden kann. Die Erzeugung von elektrischem Strom kann aber auch elektromagnetisch durch einen kleinen Magneten erfolgen, was den Vorteil hat, dass dann der Weg beim Drücken der Feder ausgenutzt werden kann. Vorzugsweise wird die elektrische Energie jedoch beim Entspannen der Feder während der Stechphase gewonnen. Die gewonnene elektrische Energie kann dann zum Beispiel für die Überprüfung der eingesaugten Probenmenge oder für die Versorgung energieautarker Sensoren verwendet werden. Der untere Teil der Hülse 17 kann nach unten verschoben werden und dadurch den Abwurf der Sensoreinheit ermöglichen.

In der in Fig. 3a gezeigten Konstruktion wird ein pneumatischer Antrieb mit vorgespannter Feder eingesetzt. Die Feder wird dabei mittels eines Dreh-, Zugoder Druckmechanismus gespannt. Beim Auslösen entsteht ein komprimierter Gasraum, welcher auf die elastische Membran 4 der Sensoreinheit wirkt.

Weiterhin besteht die Möglichkeit über eine Pumpe einen Flüssigkeitsdruck oder einen Gasdruck über einen Verdichter auf die Sensoreinheit auszuüben. Für beide Varianten ist allerdings elektrische Energie notwendig, die im einfachsten Fall über Batterien zugeführt werden müsste. Demgegenüber wird es gerade als ein Vorteil der erfindungsgemäßen Vorrichtung angesehen, dass sie ohne die Energie von Batterien auskommt.

Andere Möglichkeiten zum Antrieb der Feder bestehen darin, dass die Federkraft ohne ein Gaspolster direkt auf die elastische Membran 4 der Sensoreinheit wirkt.

Die Druckkraft kann aber auch über einen Mikromotor oder über eine Mikromotor/Getriebe-Einheit aufgebracht werden, welche direkt auf die Sensoreinheit wirkt. Die dafür notwenige Energie muss extern oder über eine elektrische Speichereinheit zugeführt werden. Dabei würde zwar die erfindungsgemäße Vorrichtung baulich größer werden, jedoch wäre der Einsatz eines Motors für Vorrichtungen mit hohem Probendurchsatz, die z.B. in Krankenhäusern zum Einsatz kommen können, durchaus sinnvoll.

Schließlich gibt es auch die Möglichkeit, über eine installierte Gasdruckpatrone im Handteil 1 durch Knopfdruck dosierte Druckstöße auf die Sensoreinheit auszuüben. Die Funktionsfähigkeit der Vorrichtung erfordert dann allerdings den gelegentlichen Austausch von Gasdruckkartuschen.

Die in Fig. 3b gezeichnete komplette Blutentnahmevorrichtung zeigt eine im Inneren der Hülse liegende Gewindespindel mit Feder 12, die nach Lösen des Auslösers 7 den Kolben 8 nach unten treibt und dabei die über der elastischen Membran der Sensoreinheit befindliche Luftsäule zusammendrückt. oder direkt auf die elastische Membran 4 einwirkt und damit die Mikronadel 3 in die Haut treibt.

Die erfindungsgemäße Vorrichtung hat vorzugsweise die Größe eines Kugelschreibers.

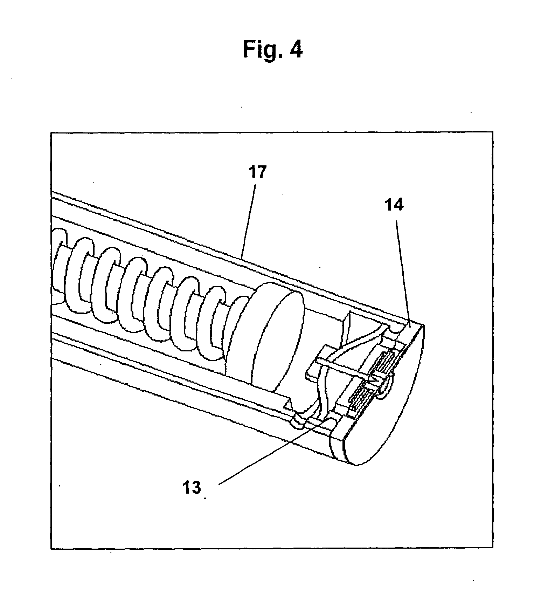

Fig. 4 zeigt Einzelheiten der Befestigung der Sensoreinheit am Handteil mit Hilfe eines Schnappverschlusses 13. Außerdem ist eine Abwurfkante 14 erkennbar, durch die die Sensoreinheit durch Verschieben einer Hülse 17 aus dem Handteil herausgedrückt werden kann.

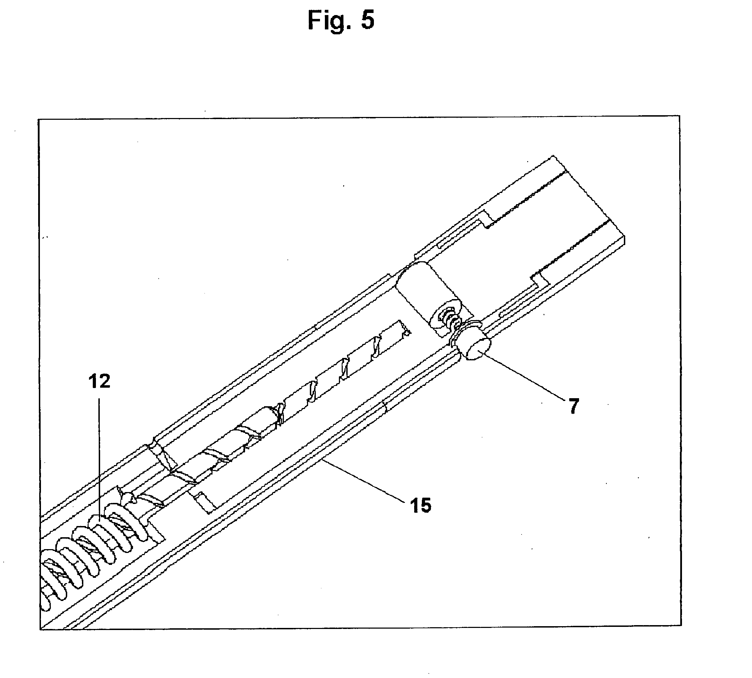

Fig. 5 zeigt einen an sich üblichen Auslösemechanismus, bei dem durch Betätigen des Auslösers 7 der Kolben frei gegeben und Druck auf die elastische Membran 4 der Sensoreinheit ausgeübt werden kann.

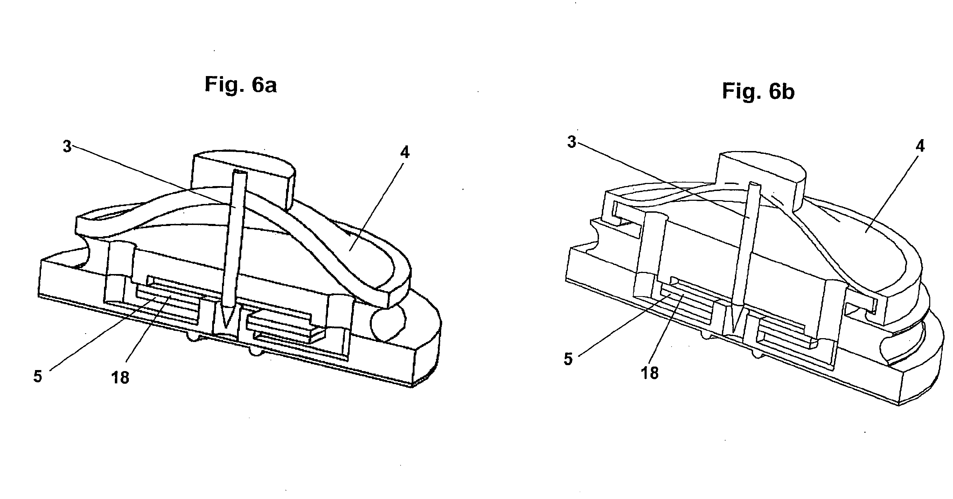

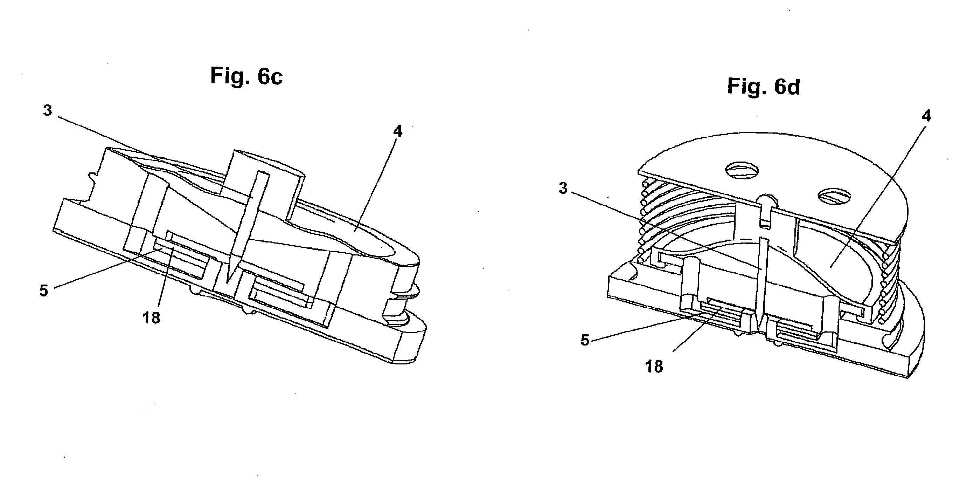

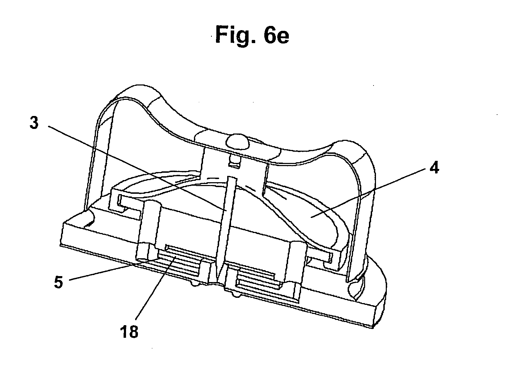

Fig. 6 a - e zeigen verschiedene Ausführungsformen für die einmal zu verwendende Sensoreinheit in Detailansichten. Die in der elastischen Membran 4 befestigte Mikronadel 3 dringt bei Einwirkung von äußerem Druck auf die elastische Membran in die Haut ein. Dabei entweicht die Luft, wobei z. B. der seitliche Ringkanal wie ein Ventil wirkt, das ein Durchtreten von Luft nur in einer Richtung zulässt. Die Nadel zieht sich beim Nachlassen des äußeren Druckes wieder zurück, wobei gleichzeitig in der Sensoreinheit ein Unterdruck entsteht, welcher das Blut in die Sensoreinheit zieht. Dort wird das Blut zunächst durch das Filtersystem 18 gefiltert, bevor es mit dem Sensorsystem 5 in Berührung kommt.

Fig. 6b zeigt eine etwas andere Ausführung der Sensoreinheit, bei der die flexible Membran in das Spritzgussteil eingerastet ist. Bei Ausübung von äußerem Druck auf die Membran entweicht die Luft aus der Sensoreinheit seitlich durch die Einrastung. Bei Entspannung der Membran wird in der Sensoreinheit ein Unterdruck aufbaut, der das Blut aus dem Gewebe saugt. Eine weitere Ausführungsform ist in Fig. 6c dargestellt, bei der eine ebene elastische Membran und eine entsprechende Vertiefung verwendet werden, die eine Variation des Volumens analog wie bei der gewölbten Membran ermöglichen.

Die Membran kann alternativ durch eine Feder oder einen Bügel in Form gehalten werden, wie es Fig. 6d und e zeigt.

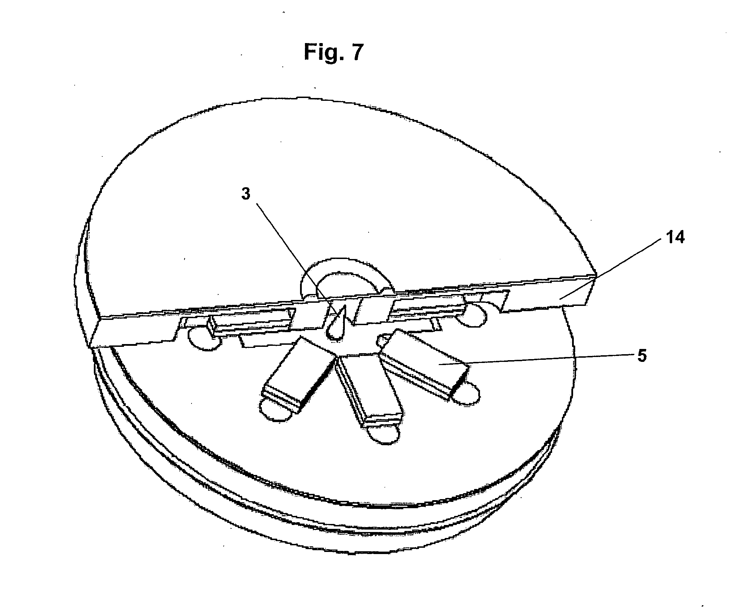

Fig. 7 zeigt eine Anordnung, in der im Sensorsystem mehrere verschiedene Nachweissysteme für unterschiedliche Analyten angeordnet sind, wobei entweder die Analyten gleichzeitig oder in einer zeitlich und räumlich vorgebbaren Reihenfolge aufgegeben werden können.

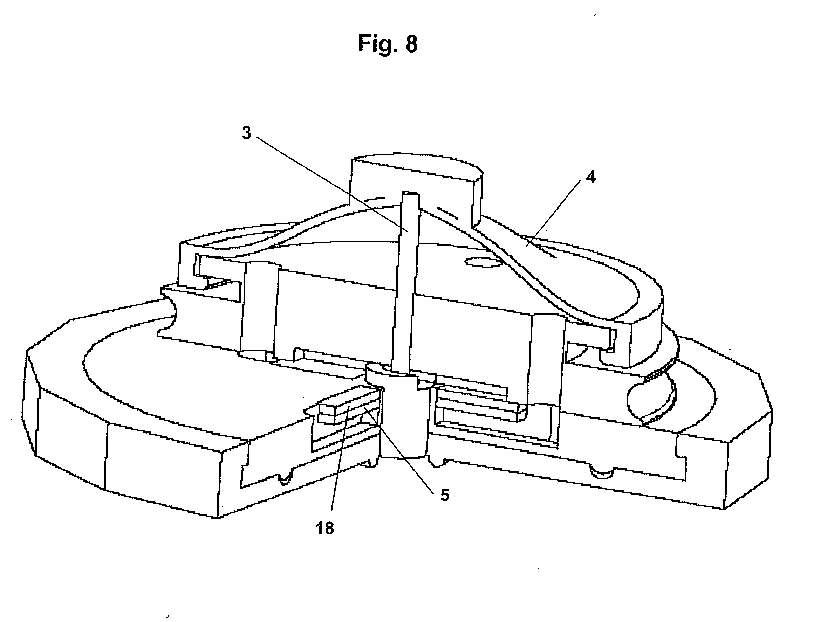

Fig. 8 zeigt eine Sensoreinheit für mehrmaligen Gebrauch. Durch einen drehbar einrastenden Blutzuführungskanal wird der Blutfluss jeweils nur auf ein Sensorsystem geleitet. Nach erfolgter Messung des Analyten dreht sich der Zuführungskanal um eine Rasterstellung weiter und öffnet den Blutkanal zum nächsten Sensorsystem. Auf diese Weise ist eine Sensoreinheit für die mehrmalige Bestimmung des gleichen Analyten einsetzbar.

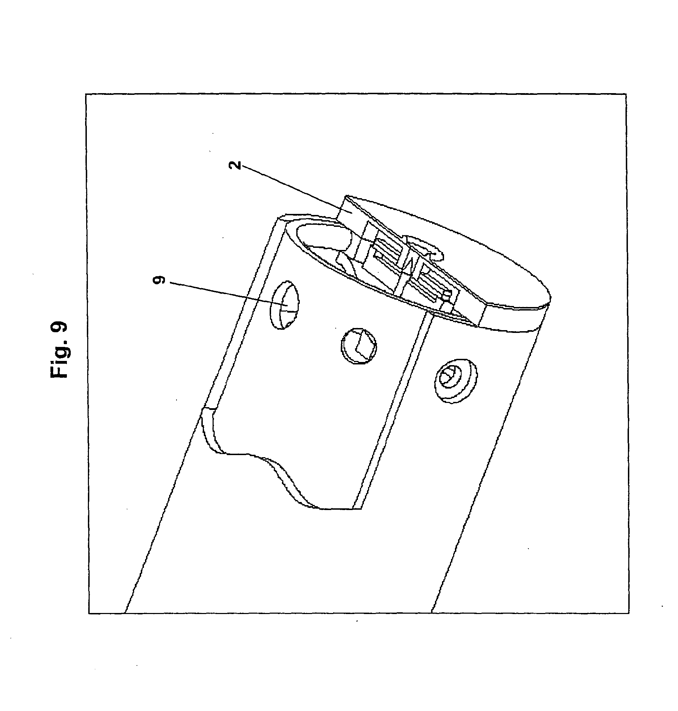

Fig.9 zeigt eine Vorrichtung zum Einstellen der Einstechtiefe durch Änderung des Luftdrucks über der elastischen Membran der Sensoreinheit durch Öffnen verschieden großer Bohrungen am unteren Handteilumfang. Eine große Öffnung lässt den Luftdruck zwischen Kolben und Membran nicht so hoch ansteigen wie eine kleinere Öffnung. Die Kraft auf die Membran und damit die Eindringtiefe der Mikronadel in die Haut lässt sich somit über den Luftdruck einstellen. Der Innenzylinder des Handteils hat verschieden große Bohrungen am Umfang. Der äußere Zylinder hat eine Bohrung. Beide Zylinder gleiten dicht aufeinander. Durch Drehung des äußeren Zylinders wird die entsprechende Innenbohrung geöffnet.

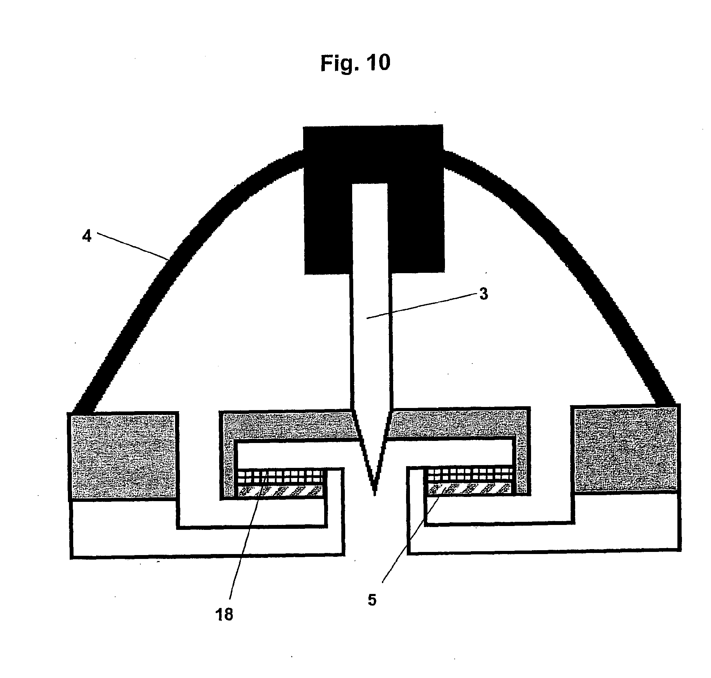

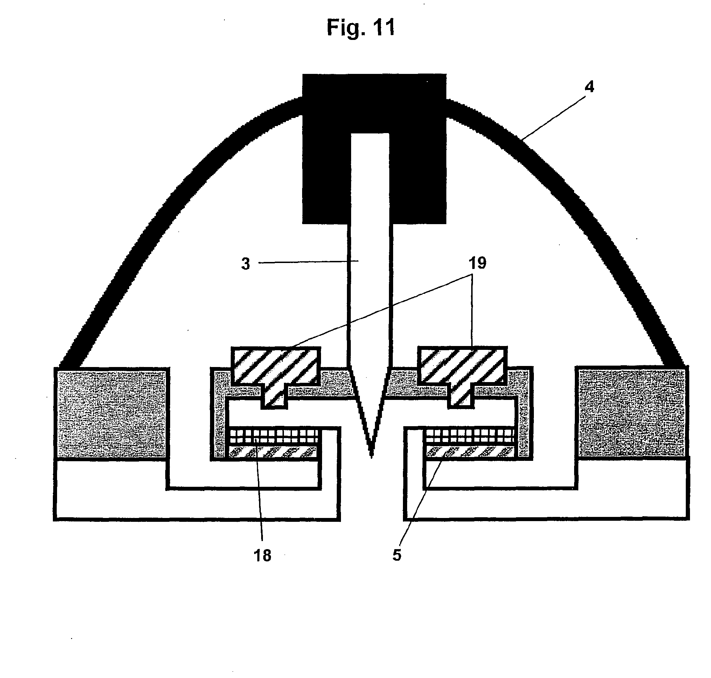

Die Fig. 10 und 11 zeigen Weiterentwicklungen der Sensoreinheit, in der das nach dem Stechen und Ansaugen der Blutprobe erfolgende Testverfahren erst nach einer entsprechenden Aufarbeitung der Probe stattfindet. Im einfachsten Fall kann es sich hierbei um eine einfache Filtration des Blutes zur Abtrennung der zellulären Bestandteile handeln. Es kann aber auch eine Filtermatrix 18 eingesetzt werden, die Substanzen zur Probenvorbereitung enthält. Das ist in Fig. 10 gezeigt.

Zusätzlich können Vorratsgefäße 19 vorgesehen sein, die Substanzen zur Vorbereitung der Blutprobe und/oder zum Spülen enthalten, wie es Fig. 11 zeigt, wobei diese Substanzen, beispielsweise durch Einwirkung von Druck über Ventile und Membranen, freigesetzt werden. In beiden Fällen kann durch eine Widerstandsmessung das korrekte Benetzen der Sensormatrix sichergestellt werden, wobei die Energieversorgung mit einem im Handteil integrierten System zur Stromerzeugung (z. B: elektromagnetisch oder mit einem Piezoelement) erfolgt.

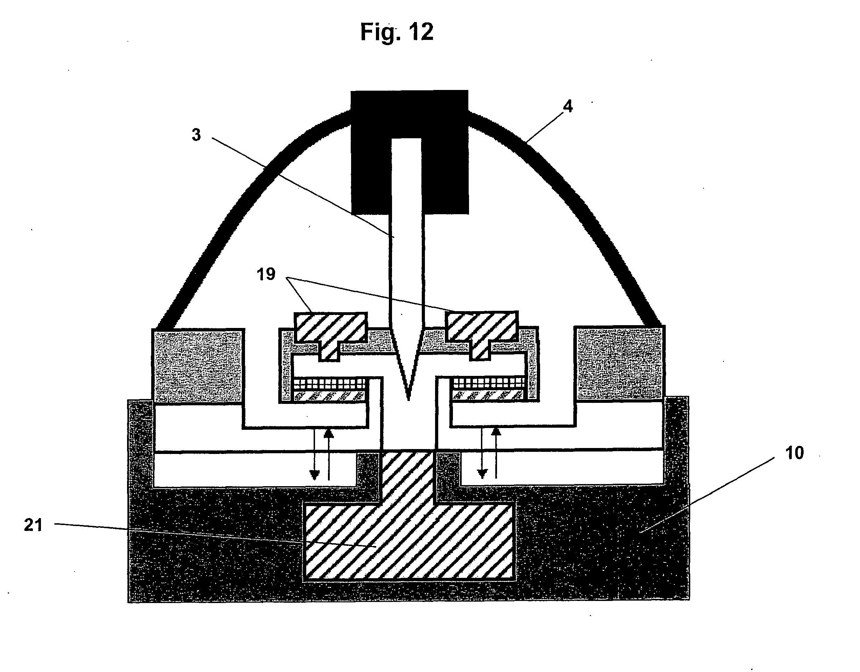

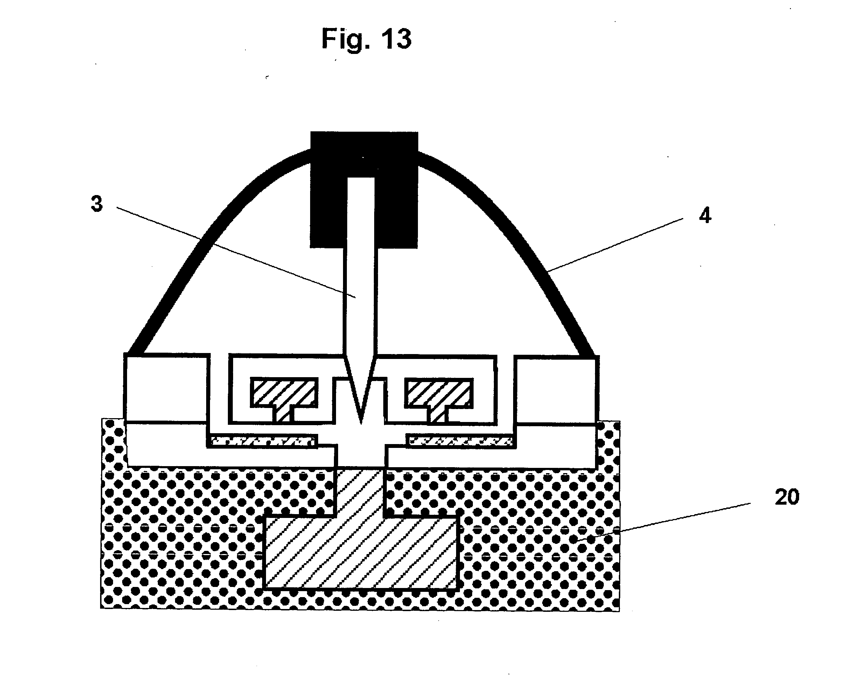

Die erforderlichen Spüllösungen und Reagenzien für die Probenvorbereitung können auch aus dem Messgerät 10 zudosiert werden, wie es Fig. 12 zeigt. Fig. 13 zeigt eine Sensoreinheit mit aufgesetzter Thermostatisierungseinheit, um Reagenzien oder Spüllösungen thermisch vorbehandeln zu können oder biochemische Reaktionen durch zyklische Temperaturänderungen zu bewirken.

Die quantitative Auswertung der im Sensorsystem erfolgenden biochemischen Reaktion kann durch optische, elektrochemische oder sonstige Sensoren oder Bestimmungsmethoden erfolgen. Als Detektionsmethoden kommen neben der Fluoreszenzmessung beispielsweise folgende optische Methoden in Betracht: die UV/Vis-Absorptionsmessung, die Surface Plasmon Resonance (SPR), die IR-Spektroskopie, die Ellipsometrie und die Colorimetrie. Als elektrochemische Messmethoden kommen die Amperometrie und die Coulometrie in Betracht. Andere Methoden wie die Surface Acustic Waves (SAW), Schwingquarze, Impedanzmessungen und Cantilever sind weitere empfehlenswerte Methoden. Besondere Bedeutung hat allerdings die quantitative Bestimmung des Analyten durch Fluoreszenzmessung.

Fig. 2g zeigt eine optische Auswertung des Sensorsystems mit Hilfe eines externen Gerätes. Die Sensoreinheit wird nach dem Ansaugen des Blutes an das externe optische Gerät gehalten, wobei die transparenten Schichten an der Sensoreinheit ein optisches Abtasten des Sensorsystems 5 ermöglichen. In analoger Weise kann eine Auswertung beispielsweise mit einem elektrochemischen Sensorsystem erfolgen.

Die Detektion des Sensorsignals ist aber auch über ein in die erfindungsgemäße Vorrichtung integriertes Messgerät möglich. Hierbei können die Messsignale über einen Transponder an ein externes Auswertegerät übermittelt werden.

Eine weitere Verfeinerung der in der erfindungsgemäßen Vorrichtung durchführbaren quantitativen Bestimmungsmethoden lässt sich dadurch erreichen, dass mehrere Blutbestandteile parallel mit unterschiedlichen Messverfahren quantitativ erfasst werden. Dazu sind in der Sensoreinheit mehrere, z. B. zwei, verschiedene Sensorsysteme 5 untergebracht, die mit gleichen oder unterschiedlichen Detektionsmethoden ausgelesen werden können. Auch wenn hierbei eine etwas höhere Blutmenge benötigt wird, ist durch die räumlich enge Verbindung von Entnahmestelle und Sensorsystemen der Blutverbrauch wesentlich kleiner als bei Standard-Methoden, bei denen eine wesentlich größere Blutmenge entnommen und zu räumlich getrennten Messgeräten gebracht werden muss. Entsprechend ist auch die Schmerzbelastung der Patienten bei Anwendung der erfindungsgemäßen Vorrichtung wesentlich geringer.

Die mit der erfindungsgemäßen Vorrichtung durchführbare Blutabnahme verbunden mit gleichzeitiger Durchführung der Nachweisreaktion von Analyten im Blut hat gegenüber den bisher bekannten Methoden den Vorteil einer weitgehend schmerzfreien Blutentnahme durch Verwendung einer Mikronadel sowie durch Verwendung von Unterdruck. Es ist außerdem als erheblicher Vorteil anzusehen, dass schon am Krankenbett ein sofortiges Ergebnis der Blutanalyse zur Verfügung gestellt werden kann. Weiterhin ist es wichtig, dass durch die nur einmal verwendbare Sensoreinheit immer eine frische, keimfreie Mikronadel zur Verfügung steht, da die Nadel steril in der Sensoreinheit eingepackt ist.

Hervorzuheben ist weiterhin, dass die erfindungsgemäße Vorrichtung ohne den Einsatz von Elektrobatterien auskommt, sondern stattdessen energieautarke Sensoren aufweist, um beispielsweise die Füllung des Sensorelementes anzuzeigen. Die in der Sensoreinheit enthaltene Mikronadel kann mit einer vorgespannten Membran abgeschlossen sein, die beim Durchstechen eine größere Öffnung freigibt, so dass sie bis unmittelbar vor dem Gebrauch völlig steril bleibt. Ebenso ist eine Bedeckung mit einer Abziehfolie möglich.

### Bezugszeichenliste:

- 1: Handteil
- 2: Sensoreinheit
- 3: Mikronadel
- 4: elastische Membran
- 5: Sensorsystem
- 6: Feder
- 7: Auslöser
- 8: Kolben
- 9: Druckausgleichsöffnung unten
- 10: Handmessgerät
- 11: Druckausgleichsöffnung oben
- 12: Gewindespindel mit Feder
- 13: Schnappverschluss
- 14: Abwurfkante
- 15: Hülse
- 16: Hülse zum Drehen
- 17: Hülse für den Abwurf der Sensoreinheit
- 18: Filtersystem
- 19: Vorratsgefäß
- 20: Heizblock
- 21: Spüllösung

## Patentansprüche

1. Vorrichtung zur Blutabnahme und gleichzeitigen Durchführung der Nachweisreaktion von Analyten im Blut, **dadurch gekennzeichnet, dass** sie aus einem Handteil (1) und einer Sensoreinheit (2) besteht, wobei
a) die Sensoreinheit (2) eine Mikronadel (3) enthält, die in einer elastischen Membran (4) befestigt bei Ausübung von Druck bzw. Kräften auf die Membran in die Haut eindringt und beim Nachlassen des Druckes oder der Kräfte die Einstichstelle unter Ausbildung eines Unterdrucks in der Sensoreinheit wieder verlässt, wodurch Blut in die Sensoreinheit gesaugt wird, welches dort mit einem Sensorsystem eine physikalische oder chemische Eigenschaftsänderung des Sensorsystems bewirkt.
b) das Handteil pneumatisch, mechanisch oder elektromechanisch auf die Membran der Sensoreinheit einwirkt.

2. Vorrichtung nach Anspruch 1, **dadurch gekennzeichnet, dass** die Sensoreinheit die Bestimmung eines oder mehrerer verschiedener Analyten im Blut erlaubt.

3. Vorrichtung nach den Ansprüchen 1 und 2, **dadurch gekennzeichnet, dass** die Sensoreinheit zum einmaligen Gebrauch bestimmt ist.

4. Vorrichtung nach den Ansprüchen 1 bis 3, **dadurch gekennzeichnet, dass** die Sensoreinheit mit Mitteln zur Filtration des Blutes ausgestaltet ist.

5. Vorrichtung nach den Ansprüchen 1 bis 4, **dadurch gekennzeichnet, dass** die Sensoreinheit Vorratsbehälter für Reagenzien und/oder Spüllösungen enthält.

6. Vorrichtung nach den Ansprüchen 1 bis 5, **dadurch gekennzeichnet, dass** die Sensoreinheit eine Einheit zum Nachweis ihrer korrekten Befüllung mit Blut und/oder Reagenzien enthält.

7. Vorrichtung nach Anspruch 6, **dadurch gekennzeichnet, dass** der Nachweis über eine Widerstandsmessung erfolgt, wobei die elektrische Energie durch mechanische Bewegung von Piezoelementen oder magnetischen Elementen erfolgt.

8. Vorrichtung nach Anspruch 1, **dadurch gekennzeichnet, dass** sie an der mit der Sensoreinheit verbundenen Seite auf ein Handmessgerät aufsetzbar ist und dort eine qualitative oder quantitative Bestimmung von einem oder mehreren Analyten durch optische oder elektrochemische oder andere Methoden erfolgen kann.

9. Vorrichtung nach Anspruch 1, **dadurch gekennzeichnet, dass** nach erfolgter Blutentnahme die Sensoreinheit abgeworfen und in der abgeworfenen Sensoreinheit mittels eines Handmessgerätes eine quantitative optische oder elektrochemische Bestimmung des Analyten erfolgen kann.

10. Vorrichtung nach Anspruch 1, **dadurch gekennzeichnet, dass** sie vor der optischen oder elektrochemischen Bestimmung der Analyten zur kontrollierten thermischen Probenvorbereitung in eine thermostatisierbare Aufnahme einsetzbar ist.

11. Vorrichtung nach Anspruch 1, **dadurch gekennzeichnet, dass** das Handteil in einer Hülse einen mit einem Auslöser arretierten und mit einer Feder gespannten, beweglichen Kolben enthält, der nach dem Auslösen die über der elastischen Membran der Sensoreinheit befindlichen Luftsäule zusammendrückt oder direkt auf die elastische Membran einwirkt und damit die Mikronadel in die Haut treibt.

12. Verfahren zur Blutentnahme und gleichzeitigen quantitativen Bestimmung von Analyten im Blut, **dadurch gekennzeichnet, dass** mittels der Vorrichtung der Ansprüche 1 bis 10 unter Ausbildung von Unterdruck Blut in die Sensoreinheit gesaugt wird, welches dort mit einem Sensorsystem eine physikalische oder chemis che Eigenschaftsänderung des Sensorsystems bewirkt.
